# EUROPEAN PATENT APPLICATION

(11) **EP 4 390 248 A1**
(43) Date of publication of application: **26.06.2024**
(21) Application number: 23217836.8
(22) Date of filing: 19.12.2023
(51) Int. Cl.: F24F 6/02, F24F 6/10, F24F 6/12, F24F 6/00

(54) **HUMIDIFIER**

(30) Priority: 19.12.2022 KR 20220177795
(71) Applicant: LG Electronics Inc., Yeongdeungpo-gu Seoul 07336 (KR)
(72) Inventor: Kim, Yongmin, Seoul 08592 (KR); Choi, Jaeheuk, Seoul 08592 (KR); Park, Dongryul, Seoul 08592 (KR); Choi, Chiyoung, Seoul 08592 (KR); Lee, Jeonghoon, Seoul 08592 (KR)
(74) Representative: Ter Meer Steinmeister & Partner

(57) **Abstract**

A humidifier includes: a heating water tank which heats water; a humidifying water tank which is connected to the heating water tank, and generates humidified air by using water supplied from the heating water tank; a first connection pipe which sends water stored in the heating water tank to the humidifying water tank; a second connection pipe which is connected such that humidified air generated in the heating water tank and the humidified air generated in the humidified water tank flow; and an exhaust pipe which discharges the humidified air existing inside the humidifying water tank to the outside.

## Description

### TECHNICAL FIELD

This invention relates to a humidifier, and more particularly, to a humidifier that generates humidified air through heating or ultrasonic vibration.

### BACKGROUND

A humidifier is an apparatus that evaporates water and discharges humidified air having high moisture content. A humidifier can generate humidified air by vaporizing water through natural evaporation, heat evaporation, or ultrasonic vibration.

Each evaporation method has advantages and disadvantages. In the case of natural evaporation, there is a problem in that a user must frequently manage the humidifying medium used.

In the case of evaporation by ultrasonic vibration, there is a problem that the humidified air may not flow actively into the indoor space as the supplied water is atomized by ultrasonic vibration, a problem that when unsterilized water is humidified, unpleasant humidified air flows into the indoor space, and a problem that ultrasonic vibrator is vulnerable to high temperature heat.

In the case of heat evaporation, safety accidents may occur if hot humidified air is discharged directly.

Korean registered patent KR 10-0158806 discloses a humidifier that humidifies water supplied from a water tank by heating and ultrasonic vibration.

However, the prior document discloses a structure in which a water heating space and a humidifying space are connected to each other. This causes a problem that the water cannot be completely sterilized when the water heating temperature is maintained below a set temperature in consideration of an ultrasonic vibrator. In addition, when water is heated to sterilize and sent to the ultrasonic vibrator, there is a problem that the ultrasonic vibrator may malfunction. That is, in the case of prior document, there is difficulty in completely sterilizing and humidifying water.

In addition, since a separate water level control device is not provided in a space for heating and a space for humidification, there is a problem in that it is difficult to directly control the water level inside a heating water tank or a humidifying water tank. In addition, since a pipe that supplies water from a water tank to the heating water tank is located below the water tank and the heating water tank, there is a problem that residual water occurs inside the pipe before and after use.

### SUMMARY

The invention has been made in view of the above problems, and may provide a humidifier that supplies comfortable humidified air.

The invention may further provide a humidifier that sterilizes water in a heating water tank, cools the heat-sterilized water, and supplies it to a humidifying water tank.

The invention may further provide a humidifier that discharges both the humidified air generated in the heating water tank and the humidified air generated in the humidifying water tank.

The invention may further provide a humidifier that uses a water tank disposed above the humidifying water tank and the heating water tank as a guide for discharging the humidified air.

The invention may further provide a humidifier that exhausts the humidified air generated in a heating water tank or the humidified air generated in a humidifying water tank through the humidifying water tank, the heating water tank, or a connection pipe.

The object is solved by the features of the independent claims Preferred embodiments are given in the dependent claims.

In accordance with an aspect of the present invention, a humidifier includes: a heating water tank which heats water; a humidifying water tank which is connected to the heating water tank, and generates humidified air by using water supplied from the heating water tank; a first connection pipe which sends water stored in the heating water tank to the humidifying water tank; a second connection pipe which is connected such that humidified air generated in the heating water tank flow into the humidified water tank; and an exhaust pipe which discharges the humidified air existing inside the humidifying water tank to the outside, so that heated water may be used to generate humidified air in the humidifying water tank or heating water tank and discharged through the exhaust pipe.

The first connection pipe and the second connection pipe may be each arranged to be vertically spaced apart, so that the area where water flows and the area where air flows in the heating water tank may be distinguished.

A length of the first connection pipe may be formed to be longer than a length of the second connection pipe, so that the temperature may be lowered as the water discharged from the heating water tank flows along the first connection pipe.

The first connection pipe may extends along a circumference of the heating water tank while being spaced apart from the heating water tank, so that the temperature may be lowered as the water discharged from the heating water tank flows along the first connection pipe.

In the second connection pipe, a first end hole may be formed at one end connected to the heating water tank.

A second end hole may be formed at the other end connected to the humidifying water tank.

The second end hole may be disposed in an upper side of the first end hole, so that humidified air generated in the heating water tank may flow into the humidifying water tank through the second connection pipe.

The first end hole may be opened in an up-down direction on an upper surface of the humidifying water tank, so that humidified air generated inside the heating water tank may flow into the second connection pipe.

The exhaust pipe may extend downward from an upper surface of the humidifying water tank.

A lower hole opened in an up-down direction may be formed at a lower end of the exhaust pipe, so that humidified air existing in the humidifying water tank can flow to the outside through the lower hole of the exhaust pipe.

A middle hole opened in a direction facing the second connection pipe may be formed in one side of a circumferential surface of the exhaust pipe, so that humidified air discharged from the heating water tank can easily flow into the exhaust pipe.

A groove, which may be recessed upwardly in a direction in which the second connection pipe may be disposed, may be formed at a lower end of peripheral wall of the exhaust pipe, so that even when the water level in the humidifying water tank is high, humidified air can flow to the inside of the exhaust pipe.

A floating valve, which may control water levels of the heating water tank and the humidifying water tank, may be disposed in the heating water tank, the first connection pipe may be disposed at a location lower than an area where the floating valve moves, and the second connection pipe may be disposed at a location higher than the area where the floating valve moves, so that the first connection pipe may be disposed at a location lower than the water level, and the second connection pipe may be disposed at a location higher than the water level.

An air supply pipe, which may supply air flowing by a blower into the humidifying water tank, may be disposed in one side of the humidifying water tank, so that the humidified air existing inside the humidifying water tank may actively flow.

The exhaust pipe may be disposed between the air supply pipe and the first connection pipe, so that air flow may be actively formed around the exhaust pipe.

On a perimeter wall of the exhaust duct, a groove may be formed from a lower end of peripheral wall to the upper side.

The groove may be disposed in a direction facing each of the second connector and the air supply duct, respectively, so that even when the water level in the humidifying water tank is high, air can flow into the exhaust pipe.

A supply pipe outlet of the air supply pipe may be opened toward a circumferential surface of the exhaust pipe.

However, since a separate hole is not formed in the direction in which the supply pipe outlet faces, the air discharged from the air supply pipe can flow the humidified air inside the humidification chamber to the exhaust pipe together.

In accordance with another aspect of the present invention, a humidifier includes: a water tank which stores water; a heating water tank which is disposed in a lower side of the water tank, and forms a heating chamber for heating water supplied from the water tank; a humidifying water tank which is disposed in a lower side of the water tank, and forms a humidifying chamber that generates humidified air by using water supplied from the heating water tank; a first connection pipe which sends water stored in the heating water tank to the humidifying water tank; a second connection pipe which is connected such that humidified air generated in the heating water tank flow into the humidified water tank flow; and an exhaust pipe which discharges water vapor generated in the heating water tank or the humidified air generated in the humidifying water tank to the lower side of the water tank, so that the water supplied from the water tank can be discharged to the lower side of the water tank as sterilized humidified air through a heating water tank and a humidifying water tank.

The humidifier further may include a discharge guider spaced apart from an outer circumference of the water tank, and an annular discharge passage through which the humidified air discharged from the exhaust pipe flows is formed between the discharge guider and the water tank, so that humidified air discharged from the humidifying water tank may flow upward along the discharge passage formed around the circumference of the water tank.

The discharge guider may include: an inner guider disposed to be spaced apart from the outer circumference of the water tank; and a lower guide wall which is disposed in a lower side of the inner guider, and may form an inclined surface toward the exhaust pipe, so that the lower guide wall can guide humidified air flowing into the exhaust pipe or guide condensate generated in the discharge passage to the exhaust pipe.

In accordance with another aspect of the present invention, a humidifier includes: a heating water tank which heats water; a humidifying water tank which is connected to the heating water tank, and generates humidified air by using water supplied from the heating water tank; a first connection pipe which sends water stored in the heating water tank to the humidifying water tank; a second connection pipe which extends from each of the heating water tank and the humidifying water tank so that humidified air generated in the heating water tank and the humidified air generated in the humidified water tank flow; and an exhaust pipe which discharges humidified air generated in the heating water tank or the humidified air generated in the humidifying water tank, wherein the exhaust pipe is connected to the second connection pipe, so that the humidified air generated in the heating water tank and the humidified air generated in the humidifying water tank may be discharged to the outside through the second connection pipe and the exhaust pipe.

A height of the second connection pipe may increase as it moves away from each of the humidifying water tank and the heating water tank, so that humidified air flowing through the second connection pipe may flow into the exhaust pipe.

The second connection pipe may be located at an uppermost end in a point of being connected to the exhaust pipe, so that humidified air flowing through the second connection pipe may flow into the exhaust pipe.

In accordance with another aspect of the present invention, a humidifier includes: a water tank, a heating water tank which heats water supplied from the water tank; a humidifying water tank which is connected to the heating water tank, and generates humidified air by using water supplied from the heating water tank; a first connection pipe which sends water stored in the heating water tank to the humidifying water tank; a second connection pipe which is connected such that humidified air generated in the humidified water tank flow into the heating water tank; and an exhaust pipe which discharges the humidified air existing inside the heating water tank to the outside, so that humidified air generated in the humidifying water tank can be discharged to the outside through the heating water tank.

### BRIEF DESCRIPTION OF THE DRAWINGS

The above and other objects, features and advantages of the present invention will be more apparent from the following detailed description in conjunction with the accompanying drawings, in which:
FIG. 1 is a perspective view of a humidifier of an embodiment of the present invention;
FIGS. 2 is a front view of a humidifier of an embodiment of the present invention;
FIG. 3 is a side cross-sectional view of a humidifier of an embodiment of the invention;
FIG. 4 a cross-sectional view for explaining the arrangement relationship of a water tank, a heating water tank, and a humidifying water tank of an embodiment of the invention;
FIG. 5 is a perspective view of a humidifying water tank, a heating water tank, a first connector, a second connector, and a water supply pipe in combination, according to an embodiment of the present invention;
FIG. 6 is an exploded view of FIG. 5;
FIG. 7 perspective view of a humidifying water tank of an embodiment of the invention;
FIG. 8 is a side view of FIG. 7;
FIG. 9 is a cross-sectional view of one side of FIG. 7;
FIG. 10 is a perspective view of a heating water tank according to an embodiment of the present invention;
FIG. 11 is a side view of FIG. 10;
FIG. 12 is a cross-sectional view of one side of FIG. 10;
FIG. 13 is a side view of a first connector of an embodiment of the present invention;
FIG. 14 is a front view of FIG. 13;
FIG. 15 is a bottom view of FIG. 13;
FIG. 16 is a cross-sectional view of one side of FIG. 13;
FIG. 17 is a side view of a second connector of an embodiment of the present invention;
FIG. 18 is a bottom view of FIG. 17;
FIG. 19 is a cross-sectional view of one side of FIG. 17;
FIG. 20 is a side view of a water supply pipe of an embodiment of the present invention;
FIG. 21 is a cross-sectional view of one side of FIG. 20;
FIG. 22 is a front view of a humidifying water tank, a heating water tank, a first connector, a second connector, and a water supply pipe in combination, according to an embodiment of the present invention;
FIG. 23 is a cross-sectional view of one side of FIG. 22;
FIG. 24 is a view showing a high water level state of the humidifying water tank and the heating water tank in FIG. 23;
FIG. 25 is a view schematically showing a connection relationship between a water tank, a heating water tank, a humidifying water tank, and a discharge guider according to a first embodiment of the present invention;
FIG. 26 is a view schematically showing a connection relationship between a water tank, a heating water tank, a humidifying water tank, and a discharge guider according to a second embodiment of the present invention; and
FIG. 27 is a view schematically showing a connection relationship between a water tank, a heating water tank, a humidifying water tank, and a discharge guider according to a third embodiment of the present invention.

### DETAILED DESCRIPTION

Advantages and features of the present invention and methods of achieving them will become apparent with reference to the embodiments described below in detail in conjunction with the accompanying drawings. However, the present invention is not limited to the embodiments disclosed below, but may be implemented in various different forms, and these embodiments are provided only to allow the invention of the present invention to be complete, and to completely inform those of ordinary skill in the art to which the present invention belongs, the scope of the invention, and the present invention is only defined by the scope of the claims. Like reference numerals refer to like elements throughout.

Expressions such as 'first', 'second', and 'third' expressed in the description are intended to distinguish the composition and do not indicate the order of composition.

Hereinafter, the present invention will be described with reference to the drawings for explaining a humidifier according to embodiments of the present invention.

### <Overall composition>

A humidifier of the present invention may discharge humidified air by using ultrasonic vibration. The humidifier of the present invention may discharge humidified air by heating water. The humidifier of the present invention may discharge humidified air generated by ultrasonic vibration and humidified air generated by heating.

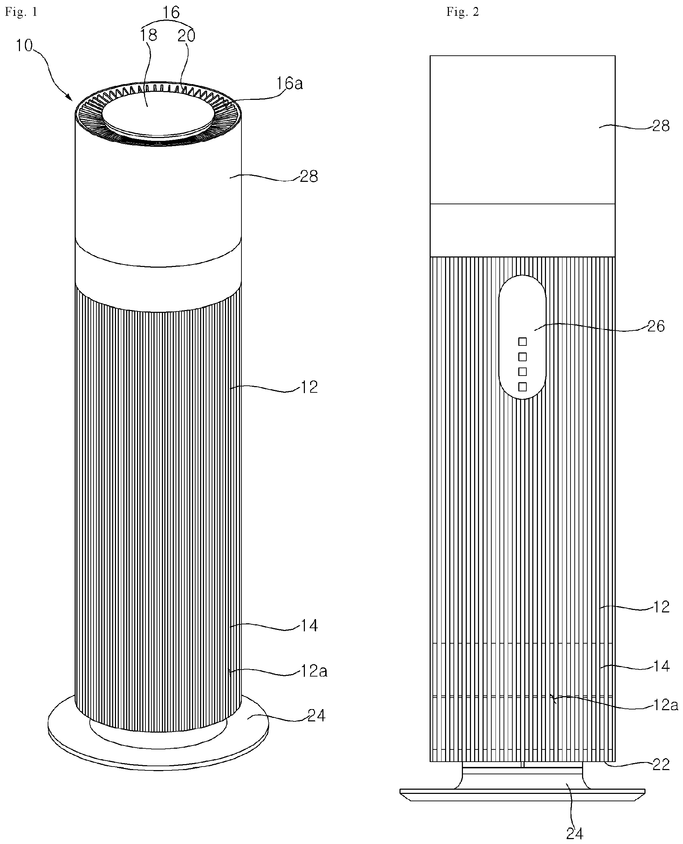

Referring to FIGS. 1 to 3, the humidifier may include a cover 10 that forms an outer shape and has an inlet 12a and an outlet 16a, a cleaning device 30 that is disposed inside the cover 10 and filters the air flowing into the inlet 12a, a blower 50 that is disposed inside the cover 10 and flows the air inside the cover 10 from the inlet 12a to the outlet 16a, a humidification device 100 that is disposed inside the cover 10 and humidifies a portion of the air flowing to the blower 50, and a discharge guider 70 that sends the air filtered by the cleaning device 30 or the air humidified by the humidification device 100 to the outlet 16a.

### <Cover>

The cover 10 may have an overall cylindrical shape.

The cover 10 may include an inlet cover 12 forming an inlet 12a through which air flows in. The cover 10 may include an outlet cover 16 forming an outlet 16a through which air is discharged.

The inlet 12a may be formed on the circumferential surface of the cover 10 having a cylindrical shape. The outlet 16a may be formed on the upper surface of the cover 10 having a cylindrical shape. The humidifier of the present invention may flow air into the circumferential surface and discharge air to the upper surface.

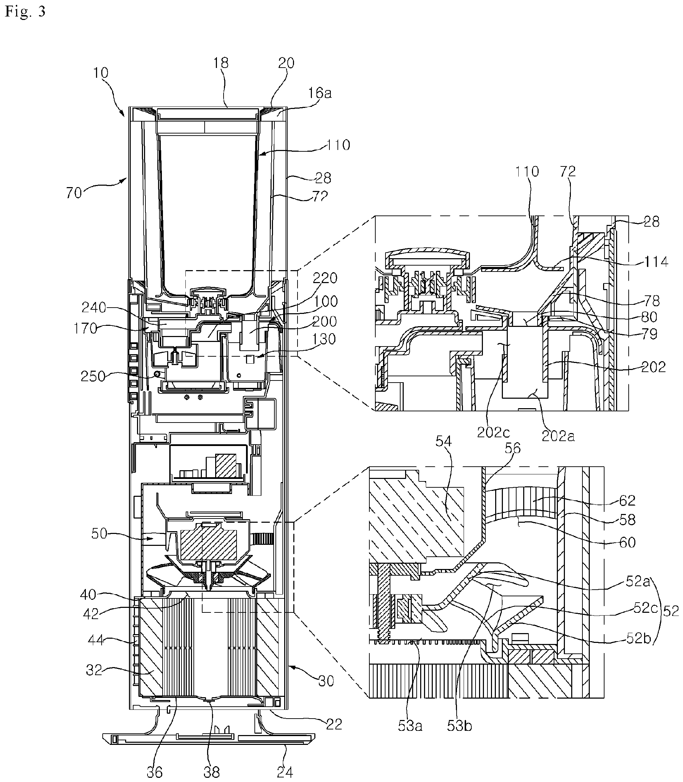

Referring to FIG. 3, the inlet cover 12 may cover the outside of the filter 32 and a blowing housing 58, which will be described below. A plurality of inlets 12a may be formed in the inlet cover 12 in the up-down direction and spaced apart in the circumferential direction. The inlet cover 12 may have a plurality of inlets 12a that are formed in the up-down direction, and spaced apart in the circumferential direction. The inlet 12a may be formed around where the filter 32 is disposed. The inlet 12a may be formed in the lower portion of the inlet cover 12.

A plurality of inlet grills 14 extending in the up-down direction are disposed in the inlet cover 12. The plurality of inlet grills 14 may be disposed to be spaced apart in the circumferential direction of the inlet cover 12. A plurality of inlets 12a may be formed between the plurality of inlet grills 14.

The inlet cover 12 may be divided into an inlet cover lower part 13b where the inlet 12a is formed and an inlet cover upper part 13a disposed in the upper side of the inlet cover lower part 13b. An input panel 26, which will be described below, may be disposed in the inlet cover upper part 13a. The inlet cover upper part 13a may cover the outer side of the blowing housing 58, and a humidifying water tank 130 and a heating water tank 170 which will be described below.

The outlet cover 16 may have a structure that is separated upward from the outer guider 28 described below. Referring to FIG. 3, the outlet cover 16 may include a water tank cover 18 that covers the upper side of the water tank 110. The outlet cover 16 may include a plurality of discharge grills 20 extending in a radial direction from the outer circumference of the water tank cover 18. A plurality of discharge grills 20 may be disposed to be spaced apart in the circumferential direction on the outer circumference of the water tank cover 18.

Referring to FIG. 1, the discharge grill 20 has a structure extending in a radial direction from the outer circumference of the water tank cover 18. A plurality of discharge grills 20 may be disposed in the upper sides of a first discharge passage 74 and a second discharge passage 76 which will be described below. The plurality of discharge grills 20 may be disposed to be spaced apart in the circumferential direction. A plurality of outlets 16a may be formed between the plurality of discharge grills 20.

The air flowing through the first discharge passage 74 and the air flowing through the second discharge passage 76 may be mixed between the plurality of discharge grills 20. In each of the plurality of discharge grills 20, the height of the outer circumferential end is formed to be higher than the height of the inner circumferential end. Accordingly, the air flowing through the first discharge passage 74 and the second discharge passage 76 may be guided inward in the radial direction.

The cover 10 may include an outer guider 28 that guides the air flowing inside to the outlet 16a. The outer guider 28 may be included in the configuration of the discharge guider 70 described below.

The cover 10 may include a lower cover 22 that covers the lower side of the inlet cover 12.

The humidifier may include a pedestal 24 that is disposed in the lower side of the cover 10 and separates the lower cover 22 from the ground by a certain distance. The upper end of the pedestal 24 may be connected to the lower cover 22. The lower cover 22 may be disposed to cover the lower surface of the humidifier spaced upward from the ground by the pedestal 24.

Referring to FIG. 2, an input panel 26 is disposed in one side of the cover 10. The input panel 26 allows a user to control the power or operation of the humidifier. A display for displaying the operating state or the like of the humidifier to a user may be disposed in the input panel 26.

### <Cleaning device>

The cleaning device 30 may filter the air introduced through the inlet 12a through the filter 32. The cleaning device 30 may flow the filtered air upward.

Referring to FIG. 3, the cleaning device 30 includes a filter 32 that filters the air flowing into the inlet 12a, and a filter mounter 34 that fixes the dislocation of the filter 32 inside the cover 10.

The filter 32 may have a cylindrical shape. Accordingly, the filter 32 may filter the air sucked from the front, rear, left, and right directions perpendicular to the up-down direction. The air introduced from the inlet 12a may flow into the inner space of the filter 32. The air passed through the filter 32 may flow to the blower 50 disposed in the upper side of the filter 32.

The filter mounter 34 includes a filter mounting plate 36 disposed in the lower side of the filter 32, a filter upper plate 40 disposed in the upper side of the filter 32, and a filter support 44 connecting the filter mounting plate 36 and the filter upper plate 40.

The filter mounting plate 36 is disposed in the lower side of the filter 32. The filter mounting plate 36 moves up and down, and may detect whether the filter 32 is disposed. A fan sterilizing device 38 that irradiates ultraviolet light upward may be disposed in the center of the filter mounting plate 36. The fan sterilizing device 38 may sterilize the inside of the blowing fan 52 or filter 32 described below.

An orifice 42 may be formed in the filter upper plate 40. The orifice 42 may be formed in the center of the filter upper plate 40. The orifice 42 may allow the air introduced to the inner side of the filter 32 to flow to the blowing fan 52. The inner circumferential end of the filter upper plate 40 has a shape bent upward, so that air flowing upward in the inner space of the filter 32 can be guided to the blowing fan 52.

The filter support 44 may connect the filter mounting plate 36 and the filter upper plate 40. The filter support 44 may be disposed to be spaced apart in the circumferential direction.

### <Blower>

The blower 50 includes a blowing fan 52 that generates airflow inside the cover 10, and a fan motor 54 that rotates the blowing fan 52.

Referring to FIG. 3, the blowing fan 52 may utilize a mixed flow fan forming a fan inlet 53a at one side and a fan outlet 53b in the opposite direction of the fan inlet 53a and in a centrifugal direction. The blowing fan 52 may include a hub 52a connected to the fan motor 54, a shroud 52b that is disposed spaced apart from the hub 52a by a certain distance and forms the fan inlet 53a, and a blade 52c extending in a radial direction to connect the hub 52a and the shroud 52b. A plurality of blades52c may be disposed to be spaced apart from each other in the circumferential direction. The fan motor 54 may be disposed in the upper side of the blowing fan 52.

The blower device includes a motor housing 56 that covers the outside of the fan motor 54, and a blowing housing 58 that is disposed to be spaced outward in a radial direction from the motor housing 56 and guides the air flowing by the blowing fan 52 upward.

A blowing passage 60 through which air flowing by the blowing fan 52 flows upward may be formed between the motor housing 56 and the blowing housing 58. An inlet cover 12 may be disposed in the outside of the blowing housing 58.

The blower 50 may include a diffuser 62 that is disposed between the motor housing 56 and the blowing housing 58 and reduces the rotational component of air flowing upward by the blowing fan 52. A plurality of diffusers 62 may be disposed to be spaced apart in the circumferential direction.

### <Discharge guider>

Referring to FIG. 3, the discharge guider 70 includes an inner guider 72 disposed to be spaced toward the outside of the water tank 110. The discharge guider 70 includes an outer guider 28 disposed to be spaced toward the outside of the inner guider 72. The outer guider 28 may be included in some components of the cover 10.

The annular second discharge passage 76 is formed between the outer guider 28 and the inner guider 72. The first discharge passage 74 having a smaller cross-sectional area than the second discharge passage 76 is formed between the inner guider 72 and the water tank 110.

The second discharge passage 76 is disposed outside the first discharge passage 74.

The discharge guider 70 includes a lower guide wall 78 disposed in the lower side of the inner guider 72. An exhaust hole 80 connected to an exhaust pipe 202, which will be described below, is formed in one side of the lower guide wall 78. Humidified air flowing from a humidifying chamber 130a may flow into the first discharge passage 74 through the exhaust hole 80.

The lower guide wall 78 is connected to the inner guider 72 at the upper side. The exhaust hole 80 may be formed at the lowest position of the lower guide wall 78. The lower guide wall 78 may form an inclined surface whose height increases toward the outside from an area where the exhaust hole 80 is formed. The upper end of the lower guide wall 78 may be formed at the same height so that it is connected to the inner guider 72.

The exhaust hole 80 is formed at a certain distance from the center of the lower guide wall 78. Accordingly, the inclination angle of the inclined surface of the lower guide wall 78 may vary depending on the direction in which it extends from the exhaust hole 80. The inclined surface formed from the area where the exhaust hole 80 is formed may vary depending on the location.

The lower guide wall 78 may allow the condensed water generated in the first discharge passage 74 to flow into the humidifying chamber 130a. The condensed water generated in the first discharge passage 74 may flow into the exhaust hole 80 along the lower guide wall 78.

The condensed water flowing into the exhaust hole 80 may flow into the humidifying chamber 130a through the exhaust pipe 202.

The water tank 110 includes a barrier 114 that protrudes outward in a radial direction from the lower end of an outer water tank 112 described below. The barrier 114 is formed along the outer circumference at the lower end of the outer water tank 112. The barrier 114 may be formed to have different degrees of protrusion from the outer water tank 112 depending on its distance from the exhaust hole 80.

### < Humidification device>

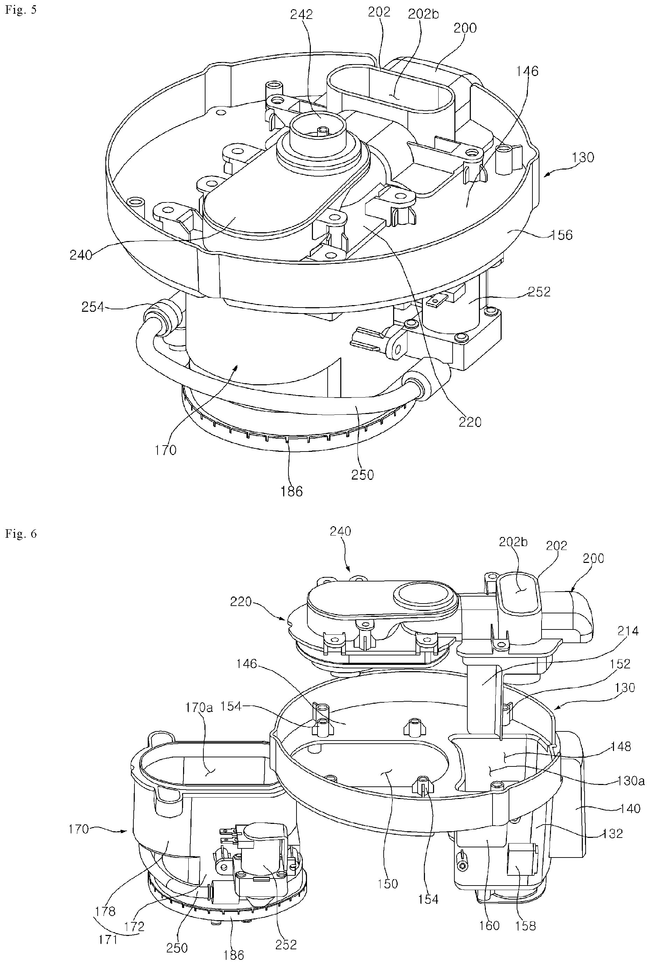

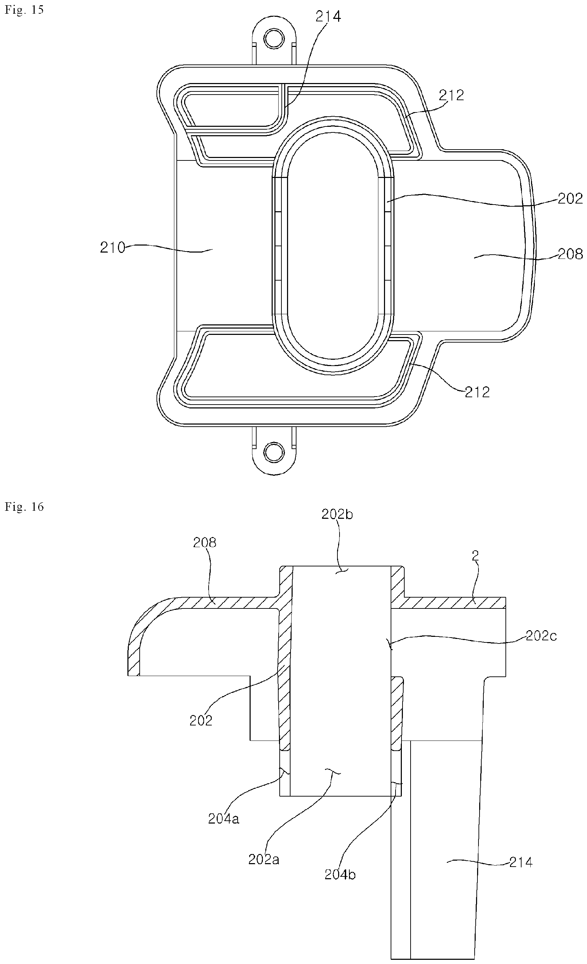

Hereinafter, a humidification device according to an embodiment of the present invention will be described with reference to FIGS. 3 to 25.

The humidification device 100 may generate humidified air by heating water stored in the water tank 110. The humidification device 100 may generate humidified air by ultrasonic vibration of water stored in the water tank 110. The humidification device 100 may heat water stored in the water tank 110 and generate humidified air by using ultrasonic vibration. The humidification device 100 may generate humidified air generated by heating water and humidified air generated by ultrasonic vibration together.

Referring to FIGS. 3 to 6, and FIG. 25, the humidification device 100 includes a water tank 110 that stores water, a heating chamber 170a that receives water stored in the water tank 110 and heats the received water, and a humidifying chamber 130a that receives water from the heating chamber 170a and humidifies the received water.

The heating chamber 170a may mean a space in which water is heated by a heater 186 described below. A space may be contained in the inside of the heating chamber 170a to form a vapor generated from water heated by the heater 186. A plurality of outer circumferences forming the heating chamber 170a may be formed.

A space of the heating chamber 170a may be formed by the heating water tank 170 and a second connector 220 which will be described below.

The humidifying chamber 130a may mean a space in which water is humidified by an ultrasonic oscillator 162a, 162b described below. A plurality of outer circumferences forming the humidifying chamber 130a may be formed. A space of the humidifying chamber 130a may be formed by the humidifying water tank 130 and a first connector 200 described below.

The heating chamber 170a and the humidifying chamber 130a may be connected to each other through two passages 230a and 250a. The humidifying chamber 130a and the heating chamber 170a may be connected to each other below an area where a floating valve 190 described below is disposed. The humidifying chamber 130a and the heating chamber 170a may be connected to each other above the area where the floating valve 190 is disposed. The two passages through which the humidifying chamber 130a and the heating chamber 170a are connected to each other may be disposed to be spaced apart from each other in the up-down direction.

The humidifying chamber 130a and the heating chamber 170a may be connected to each other in the water storage area below an area where the floating valve 190 is disposed, and be connected to each other in a different area spaced upwardly from an area where water is stored above the floating valve 190.

The heating chamber 170a and the humidifying chamber 130a may be connected to each other through a first connection passage 250a and a second connection passage 230a. The first connection passage 250a may be a passage connecting the water storage areas of each of the humidifying chamber 130a and the heating chamber 170a. The water stored in the heating chamber 170a may flow into the humidifying chamber 130a through the first connection passage 250a.

The second connection passage 230a may be a passage connecting the humidifying chamber 130a and another area spaced upwardly from the area where the water of heating chamber 170a is stored. Air in the heating chamber 170a may flow into the humidifying chamber 130a through the second connection passage 230a.

The first connection passage 250a may be formed inside a connection pipe 250 (or `first connection pipe') described below. The second connection passage 230a may be formed inside an air flow pipe 230 (or `second connection pipe') described below.

Water heated in the heating chamber 170a may be supplied to the humidifying chamber 130a through the first connection passage 250a. Vapor generated by heating water in the heating chamber 170a may flow into the humidifying chamber 130a through the second connection passage 230a. Accordingly, the humidified air discharged from the humidifying chamber 130a may be a humidified air heated through the heating chamber 170a or a humidified air heated in the heating chamber 170a and humidified in the humidifying chamber 130a. That is, the humidified air discharged through the humidifying chamber 130a may be air that is heated and sterilized through the heating chamber 170a.

The humidification device 100 includes a heating water tank 170 that is disposed below the water tank 110 and heats the water stored therein. The humidification device 100 includes a humidifying water tank 130 that humidifies the water stored therein by using ultrasonic vibration. The humidification device 100 includes a connection pipe 250 that supplies water stored in the heating water tank 170 to the humidifying water tank 130. A first connection passage 250a may be formed inside the connection pipe 250. The connection pipe 250 may use stainless steel pipe.

The humidification device 100 includes a first connector 200 that connects the humidifying water tank 130 and the internal space of the discharge guider 70. The humidification device 100 includes a second connector 220 that connects the humidifying water tank 130 and the heating water tank 170. The humidification device 100 includes a water supply pipe 240 that supplies water discharged from the water tank 110 to the heating water tank 170.

The humidification device 100 includes a water tank cover 18 that forms a space in which the humidifying water tank 130 and the heating water tank 170 are disposed. The water tank cover 18 is disposed to be spaced radially inward from the inlet cover 12. The rising air by the blower 50 may flow between the water tank cover 18 and the inlet cover 12.

The humidification device 100 includes a water tank 110 that forms a space in which water is stored and supplies water to the humidifying water tank 130 or the heating water tank 170.

The water tank 110 is disposed in the upper side of the humidifying water tank 130. The water tank 110 is disposed in the upper side of the heating water tank 170. The water tank 110 has a structure that opens upward. The water tank 110 includes a cap 118 that opens and closes the opening hole formed on the lower surface. The cap 118 is disposed in the water tank 110 to be movable up and down. When the cap 118 moves upward, the opening hole may be opened.

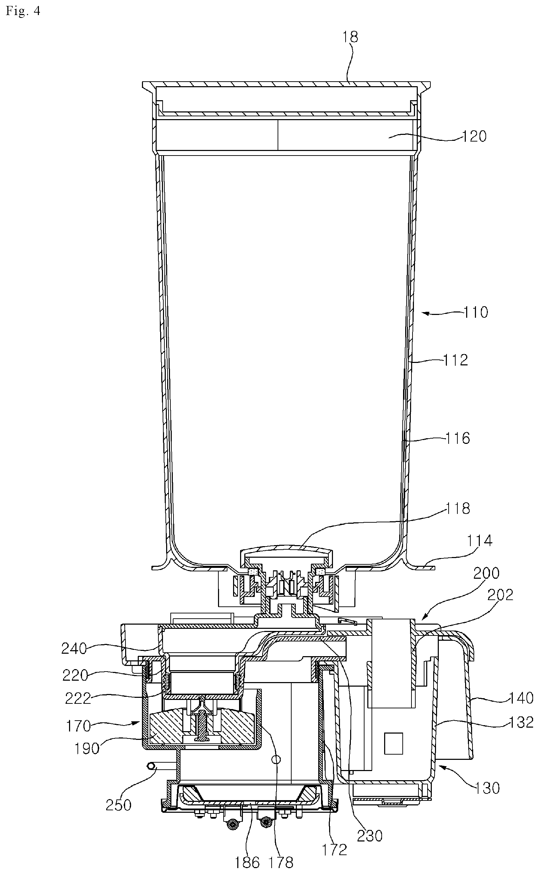

Referring to FIGS. 3 and 4, the water tank 110 of the present invention includes an outer water tank 112 and an inner water tank 116. The outer water tank 112 may be fixedly disposed in the inside of the cover 10, and may fix the dislocation of the inner water tank 116. The outer water tank 112 may guide the movement of the inner water tank 116 so that the inner water tank 116 is disposed at the upper end of the water supply pipe 240.

A barrier 114 protruding outward in the radial direction is disposed at the lower end of the outer water tank 112. The barrier 114 may be disposed in the upper side of the lower guide wall 78 so that the humidified air flowing into the first discharge passage 74 along the lower guide wall 78 may be guided to flow evenly throughout the first discharge passage 74. In the barrier 114, as it becomes closer to the exhaust hole 80, the length protruding from the outer water tank 112 becomes longer.

The inner water tank 116 moves along the outer water tank 112 and may be disposed to be coupled to a water tank connector 242 of the water supply pipe 240. The cap 118 is disposed at the lower portion of the inner water tank 116. A handle 120 may be disposed in the upper side of the inner water tank 116. A user may move the inner water tank 116 through the handle 120. The handle 120 is rotatably disposed in the inner water tank 116.

### Humidifying water tank>

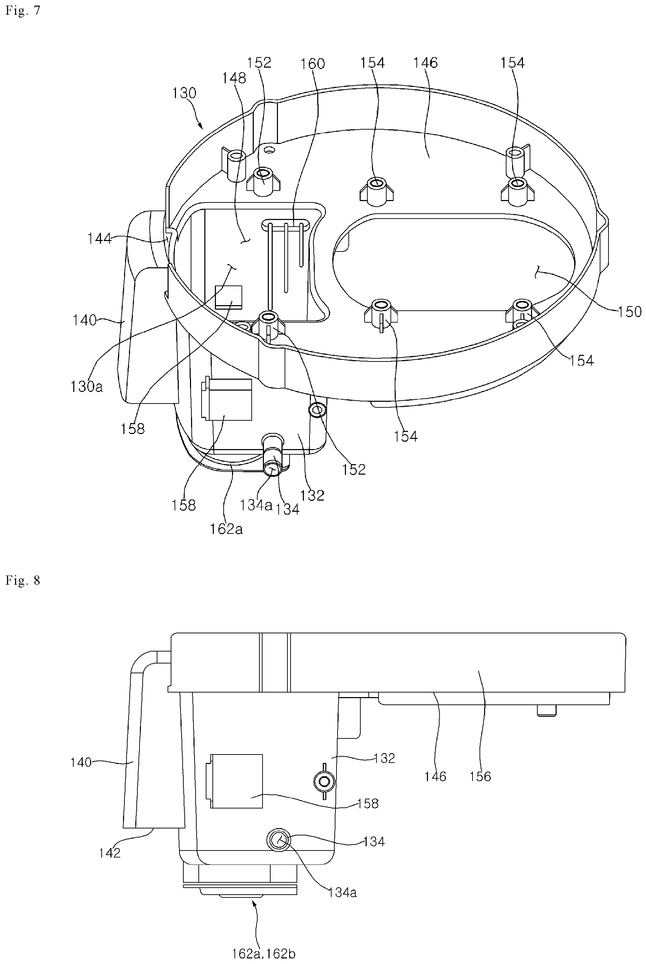

Hereinafter, the humidifying water tank of the present invention will be described with reference to FIGS. 7 to 9.

The humidifying water tank 130 includes a humidifying water tank wall 132 that forms a space in which water supplied from the heating water tank 170 is stored. The humidifying water tank wall 132 may have a cylindrical structure with an open top and a hollow interior. A humidifying chamber 130a may be formed inside the humidifying water tank wall 132.

The ultrasonic oscillator 162a, 162b may be disposed on the lower surface of the humidifying water tank wall 132 to vibrate and atomize the water stored inside the humidifying water tank wall 132. Two ultrasonic oscillators 162a, and 162b may be disposed on the lower surface of the humidifying water tank wall 132. The center of each of a pair of ultrasonic oscillators 162a and 162b may be disposed at a location overlapping a lower hole 202a or an upper hole 202b of the exhaust pipe 202. Accordingly, the humidified air that is generated by the ultrasonic oscillator 162a, 162b and flows upward may flow into the exhaust pipe 202.

Referring to FIG. 7, a water inlet hole 134a through which water is supplied to the humidifying water tank 130 is formed in one side of the circumferential surface of the humidifying water tank wall 132. Water stored in the water tank 110 may be supplied into the humidifying water tank 130 through the water inlet hole 134a. Water stored in the water tank 110 may be directly supplied to the humidifying water tank 130 through the water inlet hole 134a. In addition, water stored in the water tank 110 may be supplied to the humidifying water tank 130 through the water inlet hole 134a via the heating water tank 170. In the present invention, water heated in the heating water tank 170 is supplied to the humidifying water tank 130 through the water inlet hole 134a.

A water inlet pipe 134 protruding outside the circumferential surface of the humidifying water tank wall 132 is disposed around the water inlet hole 134a. The water inlet pipe 134 may be connected to the connection pipe 250. A mounting groove 136 into which an insertion protrusion 176 of the heating water tank 170 described below is inserted may be formed in one side of the upper end of the humidifying water tank wall 132. The mounting groove 136 may be disposed in the opposite direction to an air supply pipe 140.

Referring to FIGS. 7 to 9, in the humidifying water tank 130, the air supply pipe 140 which is disposed around one side of the humidifying water tank wall 132, and through which a portion of the air flowing by the blower 50 is supplied is disposed. In the air supply pipe 140, a supply pipe inlet 142 opened downward and a supply pipe outlet 144 opened to the humidifying chamber 130a are formed. The air supply pipe 140 may have a shape in which the cross-sectional area of the passage decreases as it progresses toward the upper side.

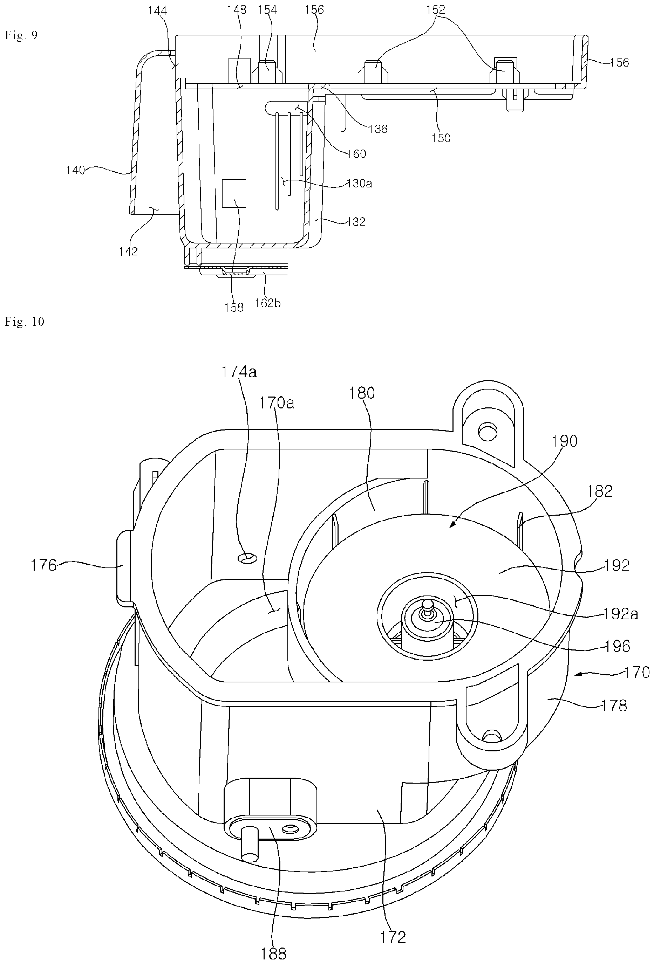

Referring to FIG. 9, the supply pipe inlet 142 is formed at the lower end of the air supply pipe 140, and the supply pipe outlet 144 is formed at the upper end of the air supply pipe 140. The supply pipe inlet 142 is opened downward. The supply pipe outlet 144 may be opened in a direction perpendicular to the supply pipe inlet 142.

The supply pipe outlet 144 may be opened from the upper side of the humidifying water tank wall 132 toward the exhaust pipe 202 described below. The supply pipe outlet 144 is disposed at a higher location than the lower hole 202a of the exhaust pipe 202.

The air supply pipe 140 may allow a portion of the air flowing upward through the blowing passage 60 to flow into the humidifying chamber 130a. The humidified air generated in the humidifying chamber 130a may quickly flow upward due to the air flowing through the air supply pipe 140.

Referring to FIG. 7, the humidifying water tank 130 may include an upper plate 146 disposed in the upper side of the humidifying water tank wall 132, and an upper peripheral wall 156 extending upward from the outer circumferential end of the upper plate 146..

The heating water tank 170, the first connector 200, and the second connector 220, which will be described below, may be connected to the humidifying water tank 130 through the upper plate 146.

Referring to FIG. 7, a first hole 148, which is opened to the upper side of the humidifying water tank wall 132, is formed in the upper plate 146. A second hole 150, which is opened upward and downward in the area where the heating chamber 170a is disposed, is formed in the upper plate 146.

The first hole 148 is a hole opened to the upper side of the humidifying chamber 130a. A first connector 200, which will be described below, may be disposed in the first hole 148.

A heating water tank 170 is disposed in the lower side of the second hole 150. A second connector 220, which will be described below, may be disposed in the upper side of the second hole 150. A first fastening protrusion 152 protruding upward may be formed in the upper surface of the upper plate 146 to be fastened to the first connector 200. A second fastening protrusion 154 protruding upward may be formed in the upper surface of the upper plate 146 to be fastened to the second connector 220.

The upper peripheral wall 156 has a structure extending upward from the outer circumferential end of the upper plate 146. A supply pipe outlet 144 of the air supply pipe 140 is formed in one side of the upper peripheral wall 156. The supply pipe outlet 144 may be formed in the upper side of the upper plate 146.

In the humidifying water tank 130, a sterilizing device 158 may be disposed in one side of the humidifying water tank wall 132. The sterilizing device 158 may be disposed in one side of the humidifying water tank wall 132 and may sterilize water stored inside the humidifying water tank wall 132. Two sterilizing devices 158 that sterilize the inside of the humidifying water tank 130 may be disposed in opposite sides of the humidifying water tank 130 of the present invention. The sterilizing device 158 may radiate ultraviolet light into the humidifying water tank wall 132.

A water level sensor 160 may be disposed in the humidifying water tank 130 to detect the level of water stored inside the humidifying water tank 130. The water level sensor 160 may be disposed in one side of the humidifying water tank wall 132. The water level of the humidifying water tank 130 may be set to be the same as the water level of the heating water tank 170. The humidifying water tank 130 and the heating water tank 170 may be connected through the connection pipe 250 to form the same water level. Therefore, it is also possible to determine the water level of the heating water tank 170 by using the water level of the humidifying water tank 130 detected by the water level sensor 160.

### Heating water tank>

Hereinafter, the heating water tank 170 of the present invention will be described with reference to FIGS. 10 to 12.

The heating water tank 170 includes a heating water tank wall 171 that stores water.

The heating water tank wall 171 includes an outer wall 172 disposed in the outer circumference. The heating water tank wall 171 includes an inner wall 178 which is disposed inside the outer wall 172, and in which the floating valve 190 is disposed.

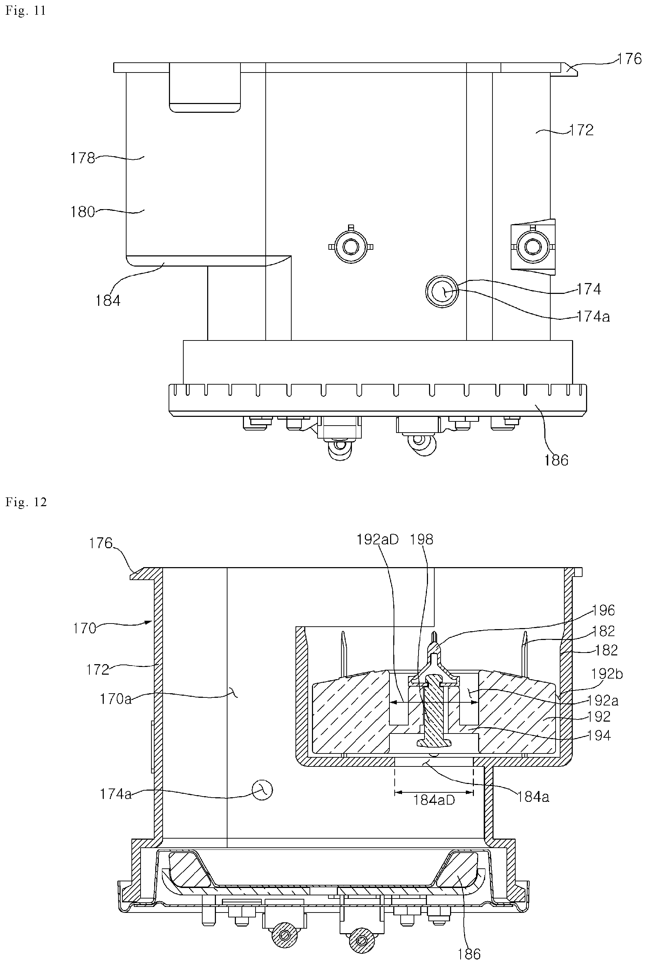

The outer wall 172 may have a cylindrical shape opened upward so that water can be stored. Referring to FIG. 12, the outer wall 172 may be hollow on the inside and have a cylindrical shape that is opened up and down. A heater 186 is disposed in the lower side of the outer wall 172. The heater 186 may be a circular sheath heater. The sheath heater may heat the lower wall of the heating water tank 170. Therefore, the lower wall of the heating water tank 170 can use a separate heating plate which is heated by a separate sheath heater.

A water discharge hole 174a through which water stored in the heating water tank 170 is discharged may be formed on one side of the outer wall 172. A water discharge pipe 174 that protrudes outward around the water discharge hole 174a may be formed in one side of the outer wall 172.

The water discharge pipe 174 may be disposed at a higher location than or at the same location as the water inlet pipe 134 formed on the humidifying water tank wall 132. The water discharge pipe 174 may be disposed to be spaced apart upward from the lower end of the heating water tank 170.

Referring to FIG. 10, an insertion protrusion 176 may be disposed in one side of the upper end of the outer wall 172. The insertion protrusion 176 may be inserted into the mounting groove 136 formed in one side of the upper end of the humidifying water tank wall 132.

A temperature sensor 188 that detects the temperature inside the heating chamber 170a is disposed in one side of the outer wall 172.

The temperature sensor 188 detects the temperature of water stored inside the heating water tank 170. The temperature sensor 188 is disposed lower than the water discharge pipe 174.

The inner wall 178 is disposed inside the outer wall 172. One circumferential surface of the inner wall 178 may be disposed to overlap one circumferential surface of the outer wall 172. The inner wall 178 includes an inner peripheral wall 180 protruding to the inside of the outer wall 172 and an inner lower wall 184 that restricts downward movement of the floating valve 190.

An inner rib 182 protruding to the inner side where the floating valve 190 is disposed is disposed in the inside of the inner peripheral wall 180. The inner rib 182 may space the floating valve 190 from the inner peripheral wall 180 by a certain distance. In addition, the inner rib 182 may prevent the floating valve 190 disposed inside the inner peripheral wall 180 from rotating in the circumferential direction.

A plurality of inner ribs 182 spaced apart in the circumferential direction may be disposed on the inner circumferential surface of the inner peripheral wall 180. The inner rib 182 may have a structure extending in the up-down direction from the inner circumferential surface of the inner peripheral wall 180.

The upper side of the inner peripheral wall 180 is opened, and the inner lower wall 184 is disposed in the lower side of the inner peripheral wall 180. The upper end of the inner peripheral wall 180 may be disposed lower than the upper end of the outer wall 172. The lower end of the inner peripheral wall 180 may be disposed higher than the lower end of the outer wall 172.

Referring to FIG. 12, a communication hole 184a is formed in the center of the inner lower wall 184. The communication hole 184a communicates the inside of the inner wall 178 and the inside of the outer wall 172. Water flowing into the inner wall 178 through the communication hole 184a may be supplied to the outer wall 172.

The center of the inner wall 178 may be formed differently from the center of the outer wall 172. Here, the center of the inner wall 178 may mean the center of the inner lower wall 184 where the floating valve 190 is disposed. In addition, the center of the outer wall 172 may mean the center of the area where the heater 186 is disposed.

The floating valve 190 may be disposed inside the inner wall 178. The height of the floating valve 190 may vary depending on the amount of water stored in the outer wall 172. The floating valve 190 may open and close a water supply hole 222a of the second connector 220 described below depending on the height.

The floating valve 190 may be disposed inside the heating water tank 170 to adjust the water level inside the heating water tank 170. The floating valve 190 may be disposed inside the heating water tank 170 to adjust the water level of the humidifying water tank 130. The water level of the humidifying water tank 130 is adjusted according to the dislocation of the floating valve 190. The floating valve 190 is disposed in the upper side of the connection pipe 250.

The floating valve 190 is disposed to be movable up and down inside the inner wall 178. The floating valve 190 has a water discharge hole 174a or a water inlet hole 134a located lower than the lowest location inside the inner wall 178.

The floating valve 190 may be located lower than the lower end of the exhaust pipe 202, at the highest location inside the inner wall 178. Accordingly, even if the water level is formed at the highest location inside the humidifying water tank 130, the water level inside the humidifying water tank 130 may be formed lower than the lower end of the exhaust pipe 202. When the floating valve 190 is located at the upper end, the water level stored in the humidifying water tank 130 may be formed lower than the lower end of the exhaust pipe 202.

Referring to FIG. 12, the floating valve 190 includes a valve body 192 formed of a material that floats on water, a valve stopper 196 that is disposed inside the valve body 192 and opens and closes the water supply hole 222a of the second connector 220 described below, and a stopper pillar 198 that is connected to the valve stopper 196 and extends downward.

The valve body 192 has a ring shape, and has a central hole 192a formed in the inside. The central hole 192a may have a shape that penetrates the center of the valve body 192. The diameter 192aD of the central hole 192a may be greater than or equal to the diameter 184aD of the communication hole 184a. Therefore, even if bubbles generated from water heated inside the outer wall 172 flows into the inner wall 178 through the communication hole 184a, they may flow to the inside of the central hole 192a to minimize the impact on the up-down movement of the floating valve 190.

A guide groove 192b corresponding to the inner rib 182 disposed on the inner circumferential surface of the inner wall 178 may be formed on the circumferential surface of the valve body 192. The guide groove 192b may be formed to extend in the up-down direction. A plurality of guide grooves 192b may be disposed to be spaced apart in the circumferential direction on the outer circumferential surface of the valve body 192.

A connecting rib 194 connected to the stopper pillar 198 is disposed on the inner circumferential surface of the valve body 192. The connecting rib 194 may have a structure that protrudes inward in a radial direction from the inner circumferential surface of the valve body 192 and is connected to the stopper pillar.

The valve stopper 196 may have a structure whose diameter decreases toward the upper side. The valve stopper 196 may have a sharp upper side. The valve stopper 196 may have a cylindrical shape with a sharp upper side. The upper end of the valve stopper 196 may be disposed higher than the upper end of the valve body 192.

### First connector>

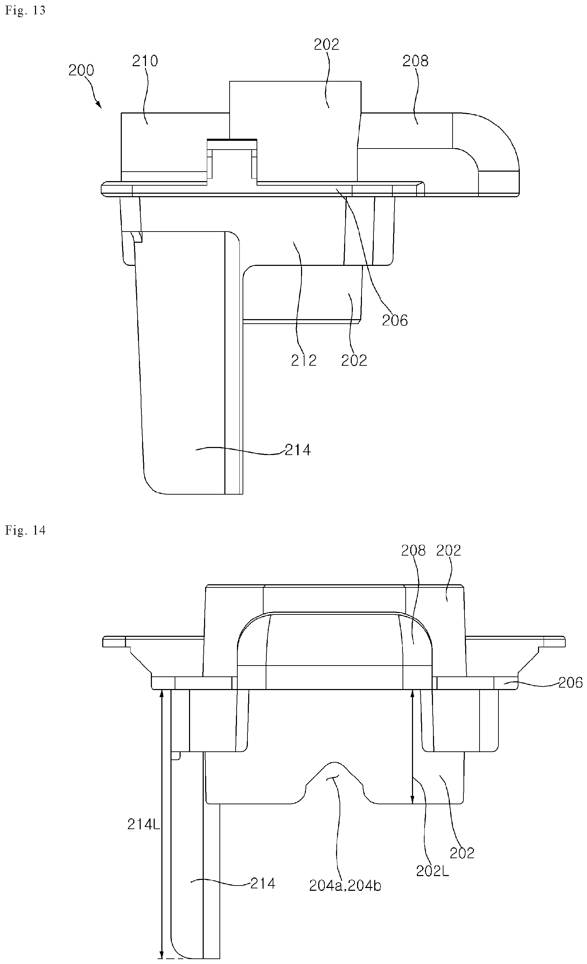

Hereinafter, a first connector according to an embodiment of the present invention will be described with reference to FIGS. 13 to 16.

The first connector 200 is connected to the upper side of the humidifying water tank 130. The first connector 200 may supply humidified air generated in the humidifying water tank 130 to a first discharge passage 74 described below. The first connector 200 may communicate the inside of the humidifying water tank 130 and the first discharge passage 74.

The first connector 200 may supply air introduced from the heating water tank 170 to the first discharge passage 74. The first connector 200 may supply air introduced through the air supply pipe 140 to the first discharge passage 74.

The first connector 200 includes an exhaust pipe 202 that communicates the humidifying chamber 130a and the first discharge passage 74. The exhaust pipe 202 has a structure that extends vertically. The lower end of the exhaust pipe 202 is disposed inside the humidifying chamber 130a. The upper end of the exhaust pipe 202 is connected to the lower guide wall 78.

The exhaust pipe 202 may have an oval cross-sectional shape. The exhaust pipe 202 may have an oval pillar shape formed long in the direction in which the two ultrasonic oscillators 162a and 162b disposed on the lower surface of the humidifying water tank wall 132 are spaced apart. Therefore, humidified air generated by the ultrasonic oscillators 162a and 162b can easily flow into the lower hole 202a of the exhaust pipe 202.

A first groove 204a, which is recessed upward in the direction in which the air supply pipe 140 is disposed, is formed in the lower end of the exhaust pipe 202. A second groove 204b, which is recessed upward in the direction in which the heating water tank 170 is disposed, is formed in the lower end of the exhaust pipe 202.

The first groove 204a and the second groove 204b may allow the air discharged from the air supply pipe 140 to easily flow into the exhaust pipe 202. The first groove 204a and the second groove 204b may allow humidified air discharged from the heating water tank 170 to easily flow into the exhaust pipe 202.

The first groove 204a and the second groove 204b may allow air flowing from the air supply pipe 140 to flow into the exhaust pipe 202, even if the humidifying water tank 130 is filled with water at a certain level. The first groove 204a and the second groove 204b may allow air flowing from the air flow pipe 230 to flow into the exhaust pipe 202, even if the humidifying water tank 130 is filled with water at a certain level. The first groove 204a and the second groove 204b are disposed in opposite directions.

The lower hole 202a is formed in the lower end of the exhaust pipe 202, and the upper hole 202b is formed in the upper end. Accordingly, humidified air generated in the humidifying chamber 130a may flow into the lower hole 202a and flow into the upper hole 202b. In the case of humidified air, due to its rising nature, it may flow from the lower hole 202a to the upper hole 202b and flow into the first discharge passage 74. However, condensed water generated in the first discharge passage 74 may flow into the upper hole 202b of the exhaust pipe 202 and flow into the lower hole 202a. Accordingly, condensed water generated in the first discharge passage 74 may flow into the humidifying chamber 130a.

A middle hole 202c may be formed in the exhaust pipe 202, in one side facing a flow pipe outlet 234 of the air flow pipe 230 of the second connector 220 described below. The middle hole 202c is disposed between the lower hole 202a and the upper hole 202b. The middle hole 202c may be disposed in the area where the humidifying chamber 130a is formed. The middle hole 202c may be disposed to face the flow pipe outlet 234 of the air flow pipe 230. Accordingly, the air flowing in the heating water tank 170 through the air flow pipe 230 may flow into the exhaust pipe 202 through the middle hole 202c. Water vapor discharged from the heating chamber 170a through the air flow pipe 230 may flow to the exhaust pipe 202 through the middle hole 202c or the lower hole 202a and then flow into the first discharge passage 74.

The first connector 200 includes a first plate 206 disposed in the upper side of the upper plate 146 of the humidifying water tank 130. The first plate 206 is disposed in the upper side of the upper plate 146. The upper plate 146 extends in a direction perpendicular to the up-down direction in which the exhaust pipe 202 extends. The first plate 206 may be disposed to be seated on the upper side of the upper plate 146. With the first plate 206 seated on the upper plate 146, the first connector 200 may be fastened to the humidifying water tank 130.

The first connector 200 includes a first connecting plate 208 disposed in the upper side of the air supply pipe 140. The first connecting plate 208 may have a shape bent upward from the first plate 206. The first connecting plate 208 may have a shape corresponding to the upper side of the air supply pipe 140.

Accordingly, the air flowing through the air supply pipe 140 may flow into the inner space of the humidifying water tank wall 132 along the first connecting plate 208. However, the air supplied through the air supply pipe 140 may flow downward inside the humidifying water tank wall 132 through the exhaust pipe 202 and flow to the exhaust pipe 202. That is, the air supplied through the air supply pipe 140 may flow inside the humidifying water tank wall 132 and flow to the exhaust pipe 202 together with the humidified air generated inside the humidifying water tank 130. This may increase the amount of humidified air flowing through the exhaust pipe 202.

The first connector 200 includes a second connecting plate 210 disposed in the upper side of the air flow pipe 230 of the second connector 220 described below. The second connecting plate 210 may have a shape bent upward from the first plate 206. The second connecting plate 210 may have a shape corresponding to the upper surface of the air flow pipe 230.

Accordingly, the air flowing through the air flow pipe 230 may flow into the inner space of the humidifying water tank wall 132 along the second connecting plate 210. The air supplied through the air flow pipe 230 may flow into the middle hole 202c of the exhaust pipe 202. In addition, the air supplied through the air flow pipe 230 may flow inside the humidifying chamber 130a and flow into the lower hole 202a of the exhaust pipe 202.

The supply pipe outlet 144 of the air supply pipe 140 and the flow pipe outlet 234 of the air flow pipe 230 may be disposed in opposite directions with respect to the exhaust pipe 202.

The first connector 200 includes a lower protrusion 212 that protrudes downward from the first plate 206, and is mounted in the inside of the humidifying water tank wall 132. The lower protrusion 212 may have a structure that protrudes downward from the first plate 206. The lower protrusion 212 may be disposed in contact with the humidifying water tank wall 132 of the humidifying water tank 130. The lower protrusion 212 contacts the inside of the humidifying water tank wall 132, so that the disposition of the first connector 200 disposed in the upper side of the humidifying water tank 130 can be fixed.

The first connector 200 may include a blocking wall 214. The blocking wall 214 extends downward from the first plate 206. The blocking wall 214 may be disposed in the area where the water level sensor 160 is disposed. The blocking wall 214 has a bending structure, and has one end and the other end that are disposed in contact with the inside of the humidifying water tank wall 132.

The length 214L of the blocking wall 214 protruding downward from the first plate 206 may be longer than the length 202L of the exhaust pipe 202 protruding downward from the first plate 206. Accordingly, the blocking wall 214 may minimize changes in the water level vibrated by the ultrasonic vibrator 162a, 162b in the area where the water level sensor 160 is disposed.

### Second connector>

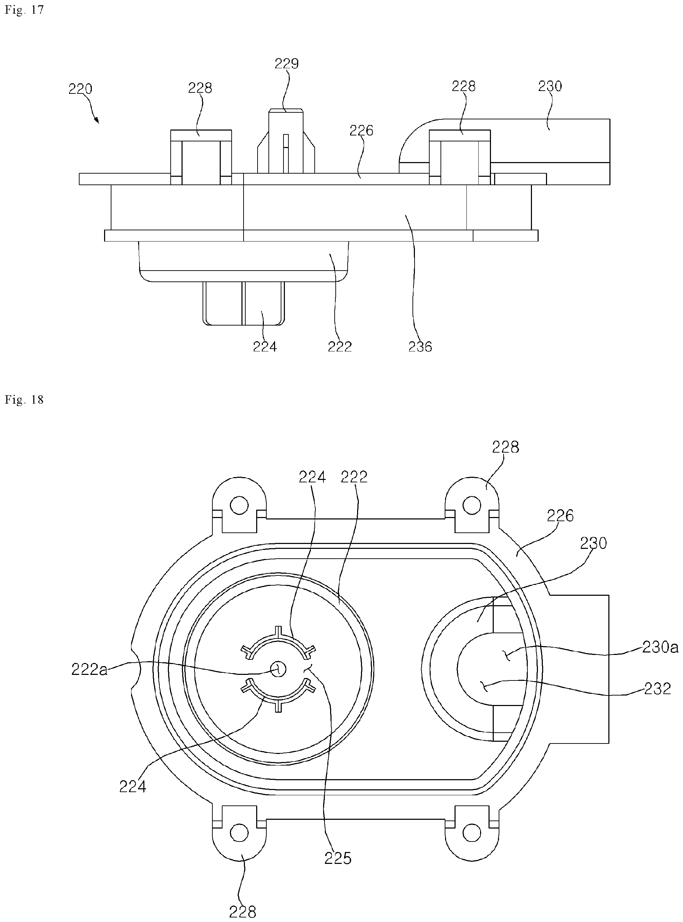

Hereinafter, a second connector according to an embodiment of the present invention will be described with reference to FIGS. 17 to 19.

The second connector 220 may cover the upper side of the heating water tank 170. The second connector 220 may supply water vapor generated in the heating water tank 170 into the humidifying water tank 130. The second connector 220 may supply water discharged from the water tank 110 to the heating water tank 170.

The second connector 220 has the water supply hole 222a through which water supplied from the water supply pipe 240 described below is supplied to the heating water tank 170. The water supply hole 222a is disposed in the upper side of the inner wall 178 of the heating water tank 170.

The second connector 220 includes a water supply tank 222 that temporarily stores water supplied from the water supply pipe 240 described below and supplies it to the heating water tank 170. The water supply tank 222 is disposed in the upper side of the inner wall 178. The water supply hole 222a, which supplies water supplied from the water supply pipe 240 into the inner wall 178, is formed in the water supply tank 222.

The second connector 220 includes a second plate 226 disposed in the upper side of the upper plate 146. The second plate 226 is disposed in the upper side of the upper plate 146 to seat the second connector 220 in the humidifying water tank 130. The second connector 220 may be fastened by a separate fastening means while the second plate 226 is mounted on the upper plate 146.

The water supply tank 222 may have a shape that is concave downward from the second plate 226. The water supply tank 222 may have a cylindrical shape that is recessed downward from the second plate 226. The diameter 222D of the internal space formed by the water supply tank 222 may be smaller than the diameter 178D of the internal space formed by the inner wall 178.

The lower end of the water supply tank 222 is disposed lower than the upper end of the inner wall 178.

The diameter 222aD formed by the water supply hole 222a may be smaller than the diameter 184aD of the communication hole 184a formed on the lower surface of the inner wall 178. The diameter 222aD formed by the water supply hole 222a may be smaller than the diameter 192aD of the central hole 192a formed in the floating valve 190. The water supply hole 222a may be disposed above the communication hole 184a. The water supply hole 222a may be formed above the central hole 192a. Therefore, the water flowing into the inner wall 178 through the water supply hole 222a may flow to the inner space of the outer wall 172 through the center hole 192a of the floating valve 190 and the communication hole 184a of the inner wall 178.

A guide protrusion 224 is formed on the lower surface of the water supply tank 222 to protrude downward. A pair of guide protrusions 224 in which semicircular structures are disposed spaced apart from each other may be disposed on the lower surface of the water supply tank 222. A pair of guide protrusions 224 are spaced apart from each other to form a gap 225. Water may be discharged through the gap formed between the pair of guide protrusions 224.

The valve stopper 196 of the floating valve 190 may be disposed inside the guide protrusion 224. The guide protrusion 224 can prevent the valve stopper 196 from vibrating in a direction perpendicular to the up-down direction when it moves up and down. The water supply hole 222a is formed inside the guide protrusion 224.

At least a portion of the guide protrusion 224 may be located in the central hole 192a formed inside the valve body 192.

The second connector 220 includes an air flow pipe 230 that sends water vapor generated inside the heating chamber 170a to the humidifying chamber 130a.

The air flow pipe 230 protrudes upward from the second plate 226, and has a structure extending in the direction in which the humidifying chamber 130a is disposed. The air flow pipe 230 includes a flow pipe inlet 232 (or "first end hole") through which air inside the heating chamber 170a flows, and a flow pipe outlet 234 (or "second end hole") through which air flows inside the air flow pipe 230 flows into the humidifying chamber 130a.

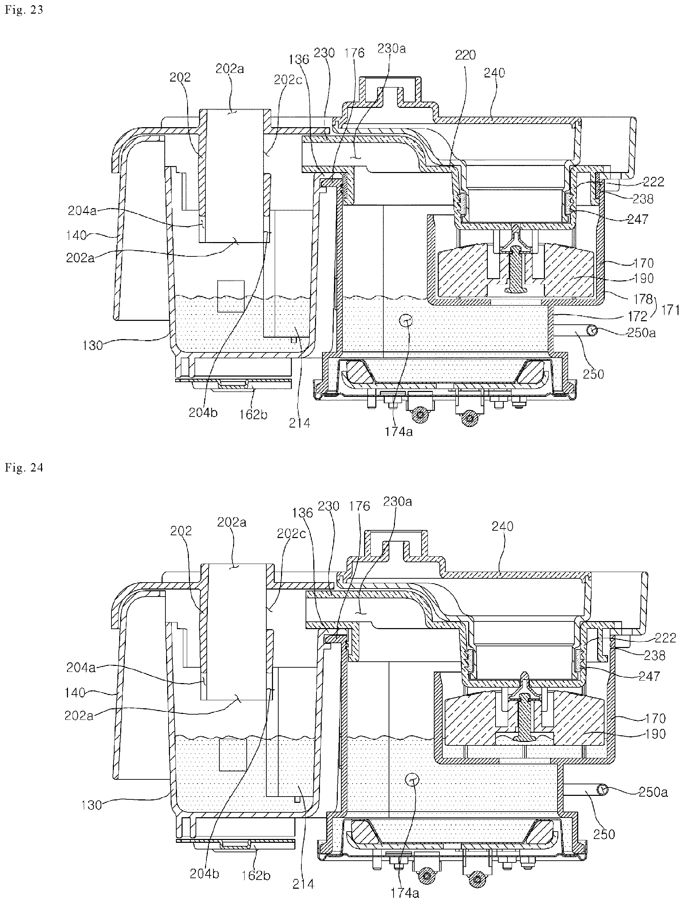

The flow pipe inlet 232 of the air flow pipe 230 is formed to be opened in the up-down direction. The flow pipe outlet 234 of the air flow pipe 230 is opened in a direction perpendicular to the flow pipe inlet 232. The flow pipe outlet 234 is opened toward the exhaust pipe 202 disposed inside the humidifying chamber 130a. Referring to FIG. 23, the flow pipe inlet 232 may be disposed not to overlap the inner wall 178 in the up-down direction. That is, the flow pipe inlet 232 may be disposed in the upper side of an area excluding an area where the inner wall 178 is disposed.

The second connector 220 includes a lower peripheral wall 236 that protrudes downward from the second plate 226. The lower peripheral wall 236 may be disposed in contact with the inner surface of the outer wall 172 of the heating water tank 170. In addition, a first sealer 238 may be disposed between the lower peripheral wall 236 and the outer wall 172 to prevent air or water vapor inside the heating chamber 170a from leaking to the outside.

A plurality of first coupling protrusions 228 coupled to the second fastening protrusions 154 of the upper plate 146 may be disposed in the second plate 226. A second coupling protrusion 229 coupled to the water supply pipe 240 may be disposed in the second plate 226.

### Water supply pipe>

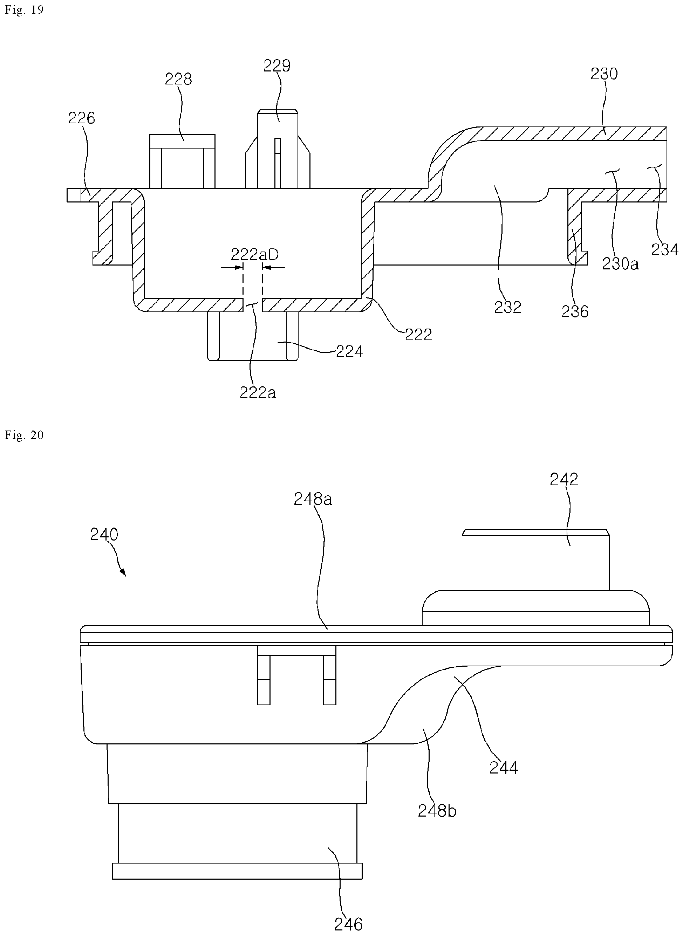

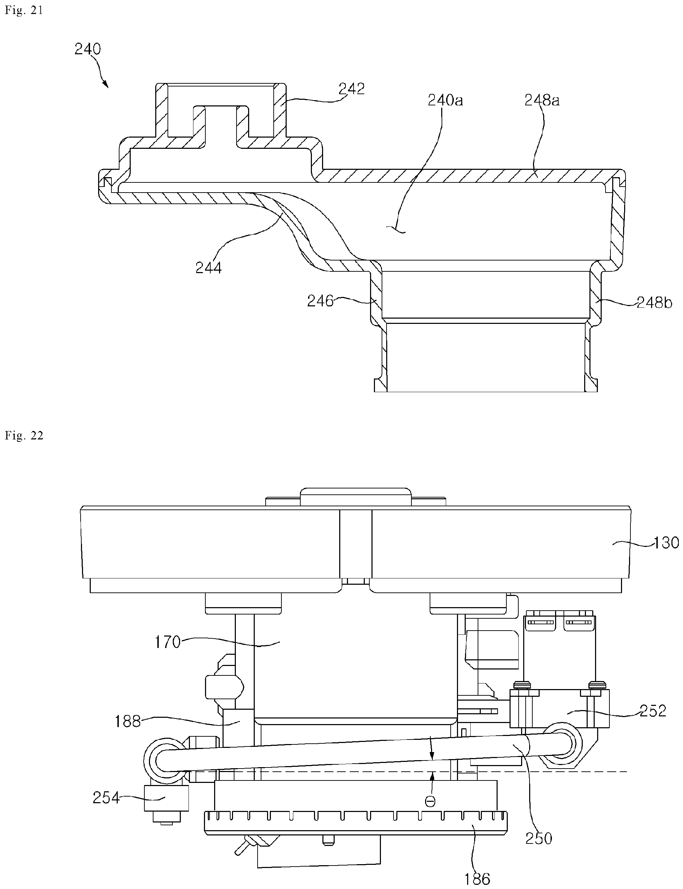

Hereinafter, a water supply pipe according to an embodiment of the present invention will be described with reference to FIGS. 20 and 21.

The water supply pipe 240 may supply water stored in the water tank 110 to the heating water tank 170. The water supply pipe 240 is disposed in the lower side of the water tank 110. The water supply pipe 240 is disposed in the upper side of the heating water tank 170. The water supply pipe 240 may form a water supply passage 240a through which water flows by gravity. The water supply passage 240a may have a bent shape.

The water supply pipe 240 includes a water tank connector 242 that is coupled to the water tank 110. The water tank connector 242 may lift the cap 118 of the water tank 110, when the water tank 110 is disposed in the upper side of the water supply pipe 240. When the water tank 110 is mounted in the upper side of the water supply pipe 240, the water tank connector 242 lifts the cap 118 so that the water stored in the water tank 110 can flow into the water supply pipe 240.

The water supply pipe 240 includes a lower supply pipe 246 which is disposed to be spaced apart from the water tank connector 242 in the up-down direction and spaced apart from the water tank connector 242 in a direction perpendicular to the up-down direction, and an upper supply pipe 244 connecting the water tank connector 242 and the lower supply pipe 246.

The center of the water tank 110 and the center of the heating water tank 170 may be disposed to be spaced apart from each other. Accordingly, the upper and lower ends of the water supply pipe 240 are disposed to be spaced apart in a direction perpendicular to the up-down direction. Accordingly, the lower supply pipe 246 is disposed to be spaced apart from the water tank connector 242 in a direction perpendicular to the up-down direction.

The lower supply pipe 246 is disposed inside the water supply tank 222 of the second connector 220. The outer circumference of the lower supply pipe 246 may be disposed in contact with the inner circumference of the water supply tank 222. A second sealer 247 may be disposed between the lower supply pipe 246 and the water supply tank 222. The second sealer 247 may allow the water flowing into the water supply tank 222 to flow only into the heating water tank 170.

The upper supply pipe 244 may have a structure extending in a direction perpendicular to the up-down direction.

Referring to FIG. 21, the water supply pipe 240 may have a structure in which two components, i.e., an upper cover 248a and a lower cover 248b, are coupled. The water tank connector 242 is disposed in the upper cover 248a. The lower supply pipe 246 is disposed in the lower cover 248b. The upper supply pipe 244 may be disposed in the area where the upper cover 248a and the lower cover 248b are coupled. The upper cover 248a and the lower cover 248b may be coupled by fusion.

### Connection pipe>

The connection pipe 250 connects the heating chamber 170a and the humidifying chamber 130a. The connection pipe 250 supplies water stored in the heating chamber 170a to the humidifying chamber 130a. The connection pipe 250 connects the water inlet pipe 134 of the heating chamber 170a and the water discharge pipe 174 of the humidifying chamber 130a. The connection pipe 250 may have a structure whose height is lowered as it progresses from the water inlet pipe 134 to the water discharge pipe 174.

In addition, the connection pipe 250 may have a structure whose height is maintained as it progresses from the water inlet pipe 134 to the water discharge pipe 174.

Referring to FIG. 22, the connection pipe 250 may form an inclination angle 0 such that its height changes as it progresses from the water inlet pipe 134 to the water discharge pipe 174.

In one side of the connection pipe 250, a connection pipe valve 252 is disposed to open and close the first connection passage 250a formed inside the connection pipe 250. The connection pipe valve 252 may use a solenoid valve. The connection pipe valve 252 may open and close the inside of the connection pipe 250 according to an electrical signal. When the passage inside the connection pipe 250 is opened by the connection pipe valve 252, water inside the heating chamber 170a may flow to the humidifying chamber 130a. The connection pipe 250 is disposed to extend along the circumference of the heating water tank 170 while being spaced apart from the heating water tank 170.

The connection pipe valve 252 may open the passage inside the connection pipe 250, when the water temperature inside the heating water tank 170 detected by the temperature sensor 188 is a set temperature or higher.

The connection pipe valve 252 may be disposed closer to the water inlet pipe 134 than the water discharge pipe 174.

An ion sensor 254 may be disposed in one side of the connection pipe 250 to measure the amount of ions contained in the water supplied to the humidifying water tank 130. The ion sensor 254 may include a structure that is connected to the connection pipe 250, and supplies water flowing from the connection pipe 250 to the humidifying water tank 130. The ion sensor 254 is connected to the connection pipe 250 at one side. The ion sensor 254 may supply flowing water to the humidifying water tank 130 through the connection pipe 250.

The connection pipe 250 may be coupled to the ion sensor 254 at one side, and connected to the connection pipe valve 252 at the other side. The connection pipe 250 may have a structure that is removable from the ion sensor 254. The connection pipe 250 may have a structure that is detachable from the connection pipe valve 252.

The ion sensor 254 may have a structure that fixes or unfixes one side of the connection pipe 250. The connection pipe valve 252 may have a structure to fix or unfix the other side of the connection pipe 250. Therefore, the connection pipe 250 may be separated from the ion sensor 252 and the connection pipe valve 252 in a simple manner.

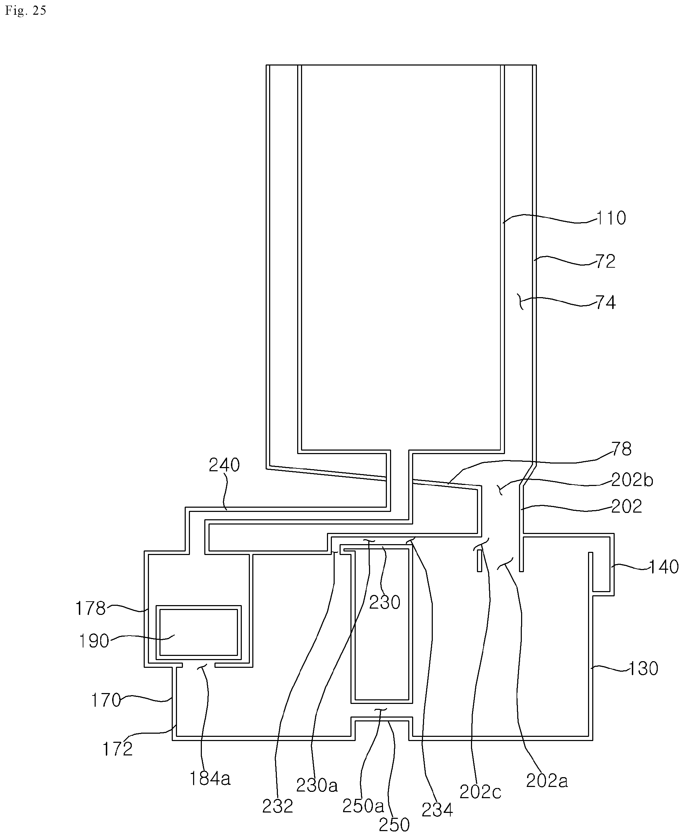

Referring to FIGS. 25 to 27, the configuration related to the flow of water or humidified air flowing from the water tank 110 to the first discharge passage 74 through the heating water tank 170 or the humidifying water tank 130 will be described.

### First embodiment>

The heating water tank 170 forming the heating chamber 170a and the humidifying water tank 130 forming the humidifying chamber 130a are connected by two connection pipes 250 and 230.

Referring to FIG. 25, the heating water tank 170 and the humidifying water tank 130 are connected to each other by a first connection pipe 250 in the space where water is stored. The heating water tank 170 and the humidifying water tank 130 are connected to each other by a second connection pipe 230 at the upper side of the space where water is stored.

The first connection pipe 250 forms a first connection passage 250a through which water flows. The first connection pipe 250 is disposed in the lower side of the inner wall 178 of the heating water tank 170. The first connection pipe 250 is disposed lower than the floating valve 190 of the heating water tank 170. The first connection pipe 250 is disposed at a location spaced lower than the lowest location of the floating valve 190.

The water inlet pipe 134 and the water discharge pipe 174 respectively connected to the first connection pipe 250 are disposed in the lower side of the inner wall 178. The water inlet pipe 134 and the water discharge pipe 174 respectively connected to the first connection pipe 250 are disposed at a location spaced lower than the lowest location of the floating valve 190.

The second connection pipe 230 forms a second connection passage 230a through which air flows. Accordingly, the inner diameter of the second connection pipe 230 is formed to be larger than the inner diameter of the first connection pipe 250.

The second connection pipe 230 is disposed in the upper side of the inner wall 178. The second connection pipe 230 is disposed in the upper side of the floating valve 190. As shown in FIG. 24, the second connection pipe 230 is spaced apart above the highest location of the floating valve 190.

The second connection pipe 230 has a first end hole 232 that communicates the inside of the heating water tank 170 and the second connection passage 230a, and a second end hole 234 that communicates the inside of the humidifying water tank 130 and the second connection passage 230a. The first end hole 232 is opened in the up-down direction. The second end hole 234 is opened toward the exhaust pipe 202.

The second connection pipe 230 is disposed above the lower end of the exhaust pipe 202. The second connection pipe 230 may supply humidified air generated inside the heating chamber 170a to the humidifying chamber 130a. The second connection pipe 230 may supply humidified air generated in the humidifying chamber 130a to the heating chamber 170a. The humidified air generated inside the heating chamber 170a or humidified air generated inside the humidifying chamber 130a may flow along the second connection pipe 230.

An exhaust pipe 202 is disposed in the humidifying water tank 130 to communicate the inside of the humidifying water tank 130 and the first discharge passage 74. The exhaust pipe 202 may send humidified air existing in the humidifying water tank 130 to the first discharge passage 74. The exhaust pipe 202 may flow condensed water generated in the first discharge passage 74 into the humidifying water tank 130.

The air supply pipe 140 is disposed in one side of the humidifying water tank 130. A portion of the air flowing by the blower 50 may flow through the air supply pipe 140. The air flowing through the air supply pipe 140 may flow to the exhaust pipe 202 via the humidifying chamber 130a. The air flowing through the air supply pipe 140 may activate air flow inside the humidifying chamber 130a. That is, the air flowing through the air supply pipe 140 may activate the humidified air existing in the humidifying chamber 130a to flow to the first discharge passage 74.

In the exhaust pipe 202, the upper hole 202b is formed in the upper side of the humidifying water tank 130, and the lower hole 202a is formed inside the humidifying water tank 130. The lower hole 202a is formed at the lower end of the exhaust pipe 202. The upper hole 202b is formed at the upper end of the exhaust pipe 202.

The upper hole 202b and the lower hole 202a may be opened in the up-down direction. In the exhaust pipe 202, the middle hole 202c is formed between the upper hole 202b and the lower hole 202a. The middle hole 202c may be formed in a direction facing the second end hole 234 of the second connection pipe 230. Accordingly, the humidified air that is discharged from the heating chamber 170a and flows through the second connection pipe 230 may flow into the exhaust pipe 202 through the middle hole 202c.

The water tank 110 is disposed in the upper side of the heating water tank 170. The water tank 110 is disposed in the upper side of the humidifying water tank 130. The water tank 110 forms the inner guider 72 and the first discharge passage 74. Accordingly, the humidified air discharged through the exhaust pipe 202 may flow upward along the circumference of the water tank 110.

The water tank 110 may have a cylindrical shape. Accordingly, the air flowing through the first discharge passage 74 may flow along an annular passage.

The center C1 of a tank hole 116a through which water is discharged from the water tank 110 is disposed to be spaced apart from the center C2 of the water supply hole 222a through which water is supplied from the heating water tank 170. The center C1 of the tank hole 116a of the water tank 110 is spaced apart from the center C3 of the upper hole 202b of the exhaust pipe 202. The center C1 of the tank hole 116a of the water tank 110 may be disposed between the center C2 of the water supply hole 222a and the center C3 of the upper hole 202b of the exhaust pipe 202.

### Second embodiment>

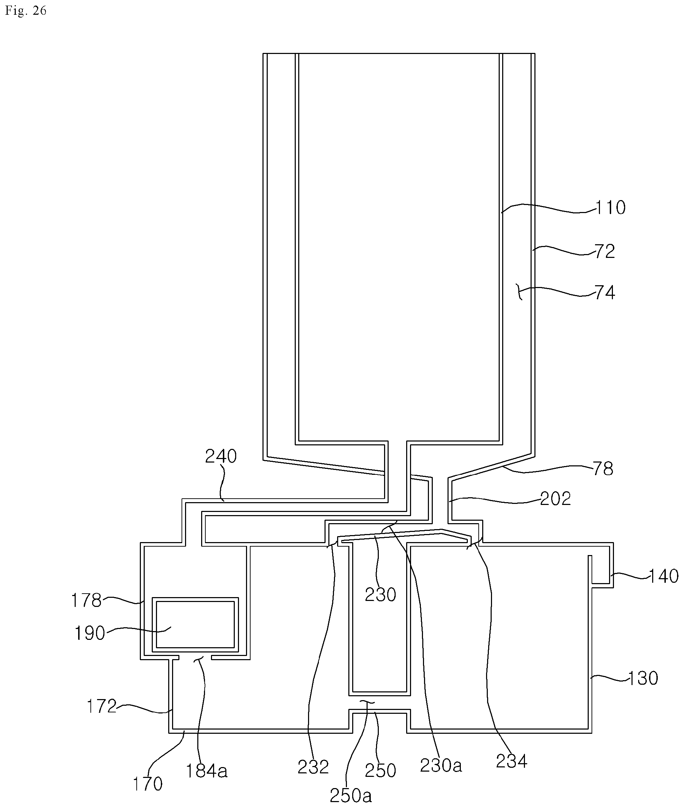

Hereinafter, a humidifier structure according to another embodiment will be described with reference to FIG. 26.

The humidifier includes a water tank 110, a heating water tank 170, and a humidifying water tank 130. The water tank 110, the heating water tank 170, and the humidifying water tank 130 may have the same structure as described in the embodiment according to FIG. 25.

The humidifier includes a first connection pipe 250, a second connection pipe 230, and an exhaust pipe 202. The first connection pipe 250 may also have the same structure as described in the embodiment according to FIG. 25.

The second connection pipe 230 connects the humidifying water tank 130 and the heating water tank 170. The second connection pipe 230 may have a bent shape at the upper side of the humidifying water tank 130 and the heating water tank 170.

An exhaust pipe 202 is connected to one side of the second connection pipe 230. The second connection pipe 230 may have a shape whose height increases as the distance from each of the humidifying water tank 130 and the heating water tank 170 increases. The exhaust pipe 202 is connected to the second connection pipe 230 at the upper end of the second connection pipe 230.

The exhaust pipe 202 may be opened so that its lower end is connected to the second connection pipe 230 and its upper end is connected to the first discharge passage 74.

The humidified air generated in the heating water tank 170 or the humidified air generated in the humidifying water tank 130 may be supplied to the first discharge passage 74 via the second connection pipe 230 and the exhaust pipe 202. Condensed water generated in the first discharge passage 74 may flow to the humidifying water tank 130 or the heating water tank 170 through the exhaust pipe 202 and the second connection pipe 230.

### Third embodiment>

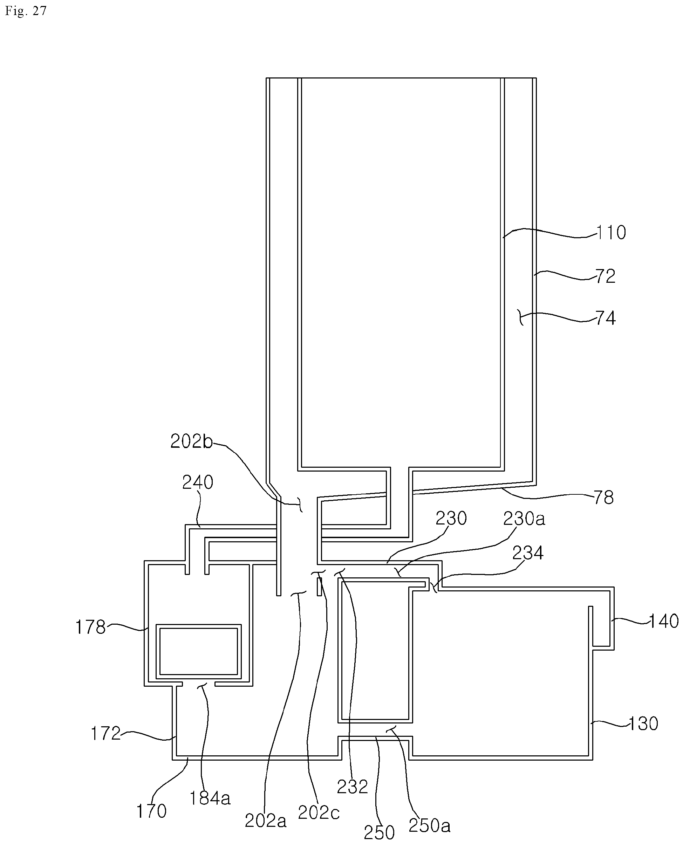

Hereinafter, a humidifier structure according to another embodiment will be described with reference to FIG. 27.

The humidifier includes a water tank 110, a heating water tank 170, and a humidifying water tank 130. The water tank 110, the heating water tank 170, and the humidifying water tank 130 may have the same structure as described in the embodiment according to FIG. 25.

The humidifier includes a first connection pipe 250, a second connection pipe 230, and an exhaust pipe 202. The first connection pipe 250 may also have the same structure as described in the embodiment according to FIG. 25.

The second connection pipe 230 may supply humidified air generated in the humidifying chamber 130a to the heating chamber 170a. The humidified air generated inside the heating chamber 170a or the humidified air generated inside the humidifying chamber 130a may flow along the second connection pipe 230.

The second connection pipe 230 has a first end hole 232 connecting the heating water tank 170 and the second connection passage 230a, and a second end hole 234 connecting the humidifying water tank 130 and the second connection passage 230a. The first end hole 232 may be opened toward the inside of the heating water tank 170. The first end hole 232 may be opened toward the exhaust pipe 202 disposed inside the heating water tank 170. The second end hole 234 may be opened in the up-down direction.

The exhaust pipe 202 may supply humidified air existing in the heating water tank 170 to the first discharge passage 74. The lower portion of the exhaust pipe 202 may be disposed inside the heating chamber 170a. The upper portion of the exhaust pipe 202 may be connected to the first discharge passage 74. The lower hole 202a of the exhaust pipe 202 may be opened in the up-down direction inside the heating water tank 170. The upper hole 202b of the exhaust pipe 202 may be opened in the up-down direction from the upper side of the heating water tank 170. The middle hole 202c of the exhaust pipe 202 may be formed in the circumferential surface of the exhaust pipe 202. The middle hole 202c may be formed at a location facing the first end hole 232 of the second connection pipe 230.

Accordingly, the humidified air generated in the heating water tank 170 may flow to the first discharge passage 74 through the exhaust pipe 202. The humidified air generated in the humidifying water tank 130 may flow into the heating water tank 170 through the second connection pipe 230. In addition, the humidified air supplied to the heating water tank 170 through the second connection pipe 230 may flow into the first discharge passage 74 through the exhaust pipe 202.

Condensed water generated in the first discharge passage 74 may flow into the heating water tank 170 through the exhaust pipe 202.

According to the humidifier of the present invention, one or more of the following effects are achieved.

First, the humidifier of the present invention can provide comfortable humidified air to indoor spaces by generating humidified air heated in the heating water tank.

Second, the water heated in the heating water tank can be supplied to the humidifying water tank through the first connection pipe, and the water heated in the heating water tank can be cooled and supplied to the humidifying water tank through the first connection pipe. This has the advantage of improving the reliability of the ultrasonic oscillator.

Third, the humidified air generated in the heating water tank can be discharged through the exhaust pipe along with the humidified air generated in the humidifying water tank. There is an advantage that the humidifying performance of the humidifier can be improved because humidified air formed in various ways is discharged together from a plurality of water tanks.

Fourth, humidified air is discharged to the lower side of the water tank, and a circular discharge passage is formed around the water tank, so that air can be discharged through the surroundings of the water tank. Since a discharge passage is formed around the water tank, a structure that can be easily separated from the water tank to the upper side can be created.

Fifth, the humidifying performance of the humidifier can be improved because the humidified air generated in the humidifying water tank and the heating water tank are mixed and discharged together.

Although the present invention has been described with reference to specific embodiments shown in the drawings, it is apparent to those skilled in the art that the present description is not limited to those exemplary embodiments and is embodied in many forms without departing from the scope of the present invention, which is described in the following claims. These modifications should not be individually understood from the technical concept or scope of the present invention.

## Claims

1. A humidifier comprising:
a heating water tank (170) configured to to heat water;
a humidifying water tank (130) connected to the heating water tank (170) , and configured to generate humidified air by using water supplied from the heating water tank (170);
a first connection pipe (250) configured to sentwater stored in the heating water tank (170) to the humidifying water tank (130);
a second connection pipe (230) connected such that humidified air generated in the heating water tank (170) flow into the humidified water tank (130); and
an exhaust pipe (202) for discharging the humidified air existing inside the humidifying water tank (130) to the outside.

2. The humidifier of claim 1, wherein the first connection pipe (250) and the second connection pipe (230) are each arranged to be vertically spaced apart.

3. The humidifier of claim 1 or 2, wherein a length of the first connection pipe (250) is formed to be longer than a length of the second connection pipe (230).

4. The humidifier of claim 1, 2 or 3, wherein in the second connection pipe (230), a first end hole (232) is formed at one end connected to the heating water tank (170), and a second end hole (234) is formed at the other end connected to the humidifying water tank (130),
wherein the second end hole (234) is disposed in an upper side of the first end hole (232).

5. The humidifier of claim 4, wherein the first end hole (232) is opened in an up-down direction on an upper surface of the humidifying water tank (130).

6. The humidifier of any one of the preceding claims, wherein the exhaust pipe (202) extends downward from an upper surface of the humidifying water tank (130),
wherein a lower hole (202a) opened in an up-down direction is formed at a lower end of the exhaust pipe (202).

7. The humidifier of any one of the preceding claims, wherein a middle hole (202c) opened in a direction facing the second connection pipe (230) is formed in one side of a circumferential surface of the exhaust pipe (202).

8. The humidifier of any one of the preceding claims, a groove (136), which is recessed upwardly in a direction in which the second connection pipe (230) is disposed, is formed at a lower end of an peripheral wall (180) of the exhaust pipe (202).

9. The humidifier of any one of the preceding claims, wherein a floating valve (190), configured to control water levels of the heating water tank (170) and the humidifying water tank (130), is disposed in the heating water tank (170),
wherein the first connection pipe (250) is disposed at a location lower than an area where the floating valve (190) moves,
wherein the second connection pipe (230) is disposed at a location higher than the area where the floating valve (190) moves.

10. The humidifier of any one of the preceding claims , wherein an air supply pipe (140), which is configured to supply air flowing by a blower (50) into the humidifying water tank (130), is disposed in one side of the humidifying water tank (170).

11. The humidifier of claim 10, wherein the exhaust pipe (202) is disposed between the air supply pipe (140) and the first connection pipe (250).

12. The humidifier of any one of the preceding claims , wherein on a perimeter wall of the exhaust duct, a groove is formed from a lower end of peripheral wall (180) to the upper side, and the groove is disposed in a direction facing each of the second connector (220) and the air supply duct (140), respectively.

13. The humidifier of claim 10, 11, or 12, wherein a supply pipe outlet (144) of the air supply pipe (140) is opened toward a circumferential surface of the exhaust pipe (202).
